(19) Europäisches Patentamt
European Patent Office
Office européen des brevets

(11) Publication number: 0 395 332 A1

(12) # EUROPEAN PATENT APPLICATION

(21) Application number: 90304332.1

(51) Int. Cl.5: A61K 7/075, A61K 7/06

(22) Date of filing: 23.04.90

(30) Priority: 25.04.89 GB 8909417

(43) Date of publication of application:
31.10.90 Bulletin 90/44

(84) Designated Contracting States:
AT BE CH DE DK ES FR GB GR IT LI NL SE

(71) Applicant: UNILEVER PLC
Unilever House Blackfriars P.O. Box 68
London EC4P 4BQ(GB)
(84) GB

Applicant: UNILEVER NV
Burgemeester s'Jacobplein 1 P.O. Box 760
NL-3000 DK Rotterdam(NL)
(84) BE CH DE DK ES FR GR IT LI NL SE AT

(72) Inventor: Kajee, Amirudeen
35 Marchwood Crescent
Woodview Phoenix, Durban 4051(ZA)
Inventor: Trevethan, Michael Albert
19 Wellington Road
Westville, Durban 3630(ZA)

(74) Representative: Tonge, Robert James et al
UNILEVER PLC Patents Division P.O. Box 68
Unilever House
London EC4P 4BQ(GB)

(54) **Hair treatment composition.**

(57) A hair treatment composition is disclosed which comprises a detergent active compound, a cationic conditioning agent, and a reducing agent which is capable of breaking disulphide bonds in the hair. The reducing agent is preferably chosen from thiols, mercaptans, sulphites, bisulphites or mixtures thereof.

The product may be a liquid shampoo composition or a solid bar product.

EP 0 395 332 A1

# HAIR TREATMENT COMPOSITION

## Background of the Invention

The present invention relates to hair treatment compositions and more particularly to conditioning hair treatment compositions which have the effect of relaxing negroid hair.

Black hair is naturally tightly curled and consequently brushing and combing the hair can be difficult. This problem is generally overcome by keeping the hair short or by subjecting the hair to treatments, for example relaxing, waving, perming, straightening or braiding. Such treatments are, however, very damaging to the hair.

Lack of brushing tends to promote poor hair condition whereas forcible brushing can cause hair breakage, resulting in African hair being generally shorter. A need emerges for a product which can make hair soft and free of tight tangle and so aids brushing.

It has also been found that washing of negroid hair causes tightening of the hair's natural curls, making it appear even shorter and causing further difficulty with brushing.

If the hair is only washed infrequently because of the above problem, the further problem of itchy scalp may arise. The hair is loaded with treatment products, pomades, curl activators, sheen sprays, etc., which results in an accumulation of debris such as dust, scales and perspiration. This provides an ideal medium for microbial growth, resulting in itchy scalp and sores.

There is therefore also a need for a product which adequately cleanses and conditions the hair without undue tightening.

Hair relaxing compositions such as those described in US 4390033 (Johnson) and US 4530830 (Revlon) are known. However, such treatments can be harsh and may damage the hair, leaving it dry and brittle. Furthermore these compositions are applied to the hair in an extra step before or after shampooing, and have to be left in contact with the hair for several minutes, sometimes up to 20 minutes, before rinsing. The procedure is time consuming, especially if the further step of conditioning of the hair is required.

## Brief summary of the Invention

It has now been found that the appearance and feel of negroid hair can be improved by the use of a conditioning hair treatment composition comprising a mild reducing agent. The hair appears softer, has more body and good combability.

Accordingly, the invention provides an aqueous conditioning hair treatment composition comprising, in addition to water

(a) detergent active compound chosen from anionic, nonionic and amphoteric detergent active compounds or mixtures thereof;

(b) cationic conditioning agent; and

(c) reducing agent capable of breaking disulphide bonds in the hair.

## Detailed Description of the Invention

### (a) Detergent active compound

The conditioning composition according to the invention comprises detergent active compound chosen from anionic, nonionic and amphoteric detergent active compounds or mixtures thereof. Preferably, the shampoo comprises an anionic detergent active compound, optionally together with one or more further detergent active compounds chosen from nonionic and amphoteric detergent active compounds and mixtures thereof.

The anionic detergent active compound is most preferably an alkyl sulphate, an alkyl ether sulphate or mixtures thereof. Commonly used anionic detergent active compounds of these kinds are $C_{10}$ to $C_{18}$ alkyl sulphates, and $C_{10}$ to $C_{18}$ alkyl ether sulphates containing 1 to 5, preferably 2 or 3 moles of ethylene oxide. These detergents are generally employed in the form of their sodium, potassium, ammonium or mono-, di- or tri-ethanolamine salts. Examples of these detergents are sodium lauryl sulphate, ammonium lauryl

sulphate, mono-, di- and tri- ethanolammonium lauryl sulphates, sodium lauryl ether sulphate (2EO), sodium lauryl ether sulphate (3EO), potassium lauryl ether sulphate (2EO) and ammonium lauryl ether sulphate (3EO).

Further suitable anionic detergent active compounds include dialkyl sulphosuccinates having the structure:

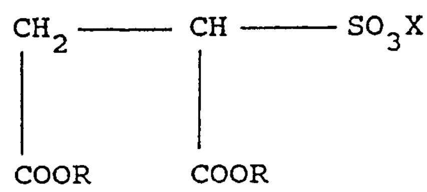

where R represents the same or different straight chain or branched chain alkyl groups having from 4 to 12 carbon atoms;

X is a solubilising cation chosen from alkali metal, ammonium, substituted ammonium and magnesium.

Particularly preferred dialkyl sulphosuccinates include those where R represents $C_6$ and/or $C_8$ alkyl groups.

Still further suitable anionic detergent active compounds include -olefin sulphonates, alkyl sarcosinates, alkyl monoglyceride sulphates, alkyl monoglyceride sulphonates, alkyl benzene sulphonates, monoalkylether sulphosuccinates, alkyl ether carboxylates, acyl isethionates and acyl methyl taurides.

The anionic detergent active compound may also be a soap. Soaps are generally defined as the salts of fatty acids, preferably the sodium, potassium or alkanolamine salts. Suitable fatty acids are the $C_{10-18}$ fatty acids which may be saturated or unsaturated.

An example of a nonionic detergent active compound is the reaction product of a sorbitan monolaurate or a sorbitan monococoate with from 20 to 80 moles of ethylene oxide and a $C_8$ to $C_{18}$ ethoxylated fatty alcohol having from 10 to 50 moles of ethylene oxide.

Examples of amphoteric detergent active compounds include amine oxides, betaines, sulphobetaines, and imidazolines.

The amount of detergent active compound present in the composition of the invention will depend on the product form. For example when the composition is a propellant-free liquid or gel intended to be poured, pumped or otherwise dispensed from a bottle or similar container or from a sachet, then the composition will generally contain from 2 to 30%, preferably from 10 to 20% by weight of detergent active compound. When, however, the composition is in the form of a liquid or gel containing a gaseous liquefiable propellant, intended to be dispensed from an aerosol can, generally as a mousse, then the concentrate, that is the part of the product without propellant, will generally contain from 2 to 40%, preferably from 10 to 30% by weight of detergent active compound.

The compositions of the invention will generally be in the form of liquid or gel shampoo composition, but may also suitably be in the form of a bar product which comprises soap or non-soap detergent.

Having regard to the product form, it can be stated generally that shampoos containing less than about 2% by weight of detergent active compound are likely in use to produce insufficient lather to clean the hair efficiently, while shampoos containing more than 40% by weight of detergent active compound may be too thick to apply conveniently to the hair and may also degrease the hair to an undesirably excessive degree.

The individual amounts of anionic detergent active compound, and nonionic and amphoteric detergent active compound, when present, will fall within limits for total detergent active compound as defined herein.

Accordingly, when only anionic detergent active compound is present, then it will comprise from 2 to 40%, preferably from 4 to 20% by weight of the composition. When a nonionic detergent active compound is present, then it will normally comprise up to 20%, preferably from 1 to 10% by weight of the composition. When the amphoteric detergent active compound is present, then it will normally comprise up to 20%, preferably from 1 to 10% by weight of the composition.

(b) Cationic Conditioning agent

The conditioning composition according to the invention also comprises cationic conditioning agents chosen from cationic detergent active compound, cationic polymers, cationic protein derivatives or mixtures thereof.

Examples of cationic detergent active compounds include

Cetyl trimethylammonium chloride
Stearyl dimethylbenzyl ammonium chloride
Cetylpyridinium chloride
Quaternium -5
Quaternium -31
Quaternium -18
and mixtures thereof

Suitable cationic polymers include

Guar Hydroxypropyltrimonium chloride Quaternium-19 Quaternium-23 Quaternium-40 Quaternium-57 Poly-(dimethyldiallylammonium chloride) Poly(dimethylbutenyl ammonium chloride)-$\alpha,\omega$-bis(triethanolammonium chloride) Poly(dipropyldiallylammonium chloride) Poly(methyl-$\beta$-propaniodiallylammonium chloride) Poly-(diallylpiperidinium chloride) Poly(vinyl pyridinium chloride) Quaternised poly (vinyl alcohol) Quaternised poly (dimethylaminoethylmethacrylate) and mixtures thereof.

Suitable cationic protein derivatives include lauryl dimonium hydroxy propylamino hydrolysed animal protein, available commercially under the trade name LAMEQUAT L, and hydrolysed keratin containing sulphur-bearing amino acids, available commercially under the trade name CROQUAT WKP.

The amount of cationic conditioning agent in the conditioning composition according to the invention is suitably from 0.001 to 10% by weight, preferably from 1 to 5% by weight.

(c) Reducing agent

The conditioning composition according to the invention also comprises a reducing agent which is capable of breaking disulphide bonds in the hair to give a mild relaxation of the curls in the hair.

Suitable reducing agents include thiols or mercaptans as well as sulphites and/or bisulphites and sodium hydroxide. Preferred examples are thioglycollic acids thiolactic acid, cysteine, thioglycerol, thioglycerolic hydrazide, thioglycolamide, glycerol monothioglycolate, beta-mercapto-propionic acid, N-hydroxyethyl mercapto-acetamide, N-methyl mercapto-acetamide, beta-mercapto-ethylamine, beta-mercapto-propionamide, 2-mercapto-ethanesulphonic acid, dimercapto-adipic acid, dithiothreitol, homocysteinethiolactone, cysteine derivatives and polythiol derivatives formed by the addition of cysteamine onto a maleic anhydride-alkylvinyl ether copolymer.

The sulphites and bisulphites which can be used are those normally used in hair waving such as the sodium and ammonium salts.

The preferred reducing agent is sulphite and/or bisulphite, in which case air oxidation is sufficient because of the short contact time between the hair and the reducing agent. This results in a single operation conditioning composition, which has the effect of relaxing the hair.

The amount of reducing agent in the conditioning composition according to the invention is suitably from 0.1 to 15% by weight, preferably from 0.2 to 10% by weight, most preferably from 0.4 to 8% by weight.

The product is preferably in the form of a conditioning shampoo which is left on the hair for a shorter time than a normal cold-waving lotion, and consequently fewer disulphide bonds are broken due to the presence of the reducing agent. This gives the effect of "relaxation" of the hair which facilitates combing and decreases hair breakage. The mild relaxing will detangle the hair, making it appear longer.

Other Ingredients

The composition of the invention can also include appropriate amounts of other conventional ingredients which can be employed in shampoos; these include for example foam boosters, further conditioning agents such as silicones, thickener, opacifiers, perfumes, colouring agents, preservatives, proteins and agents for adjusting pH, usually to a value in the range of from 4 to 8, preferably from 5.5 to 7.5, most preferably from 6.5 to 7.4.

When reducing agents such as thioglycollic acid are used in the conditioning shampoo according to the invention, it may be necessary to employ neutralising/oxidising agents. These may suitably be applied in a separate hair conditioning composition.

Use of the Conditioning Composition

The conditioning composition according to the invention can be employed in the washing and conditioning of human hair according to conventional shampooing techniques. The washing of hair using a shampoo of the invention can accordingly include the steps of pre-wetting the hair with water, adding to the hair an appropriate quantity of from about 1 to 10 ml of the shampoo, massaging the hair in order to provide a creamy lather thereby to enhance the distribution of the shampoo on the hair and to improve the cleaning thereof. The shampoo is then left on the hair for a short time, preferably for 30 seconds to 5 minutes, in order to allow the reducing agent to act on the disulphide bonds in the hair. The hair may then be rinsed and styled as desired.

The invention is further illustrated by the following Examples.

EXAMPLES

| Example 1 | |
|---|---|
| | %wt |
| JAGUAR C13S | 1.0 |
| Cocamidoproyl betaine | 8.0 |
| Triethanolamine | 0.20 |
| Lactic acid | 0.50 |
| Sodium lauryl ether sulphate | 30.0 |
| LAMEQUAT L | 2.0 |
| GAFQUAT 755N | 1.0 |
| Urea | 18.0 |
| Ammonium Bisulfite | 8.325 |
| Ammonium Sulfite | 2.0 |
| Ammonium hydroxide (32%) | 0.875 |
| Perfume | qs |
| Dye | qs |
| Water | to 100 |

JAGUAR C13S (trade name) is guar hydroxypropyltrimonium chloride,
LAMEQUAT L (trade name) is lauryl dimonium hydroxypropylamine hydrolysed animal protein, and
GAFQUAT 755N (trade name) is polyquaternium 11.

The shampoo composition of Example 1 and a placebo composition having the same formulation as Example 1 but without ammonium bisulphite, ammonium sulphite or urea were tested using a blind comparison test. A panel of 25 people (20 effective respondents) having untreated (virgin) negroid hair were given either Example 1 or Placebo to use over a period of two weeks.

Panel member were interviewed after using the sample for two weeks, and the results are as follows:

| 1. Wet Combing | | | | | |
|---|---|---|---|---|---|
| | Poor | Fair | Moderate | Good | Excellent |
| Placebo | 20% | 45% | 20% | 15% | - |
| Example 1 | 5% | - | 30% | 65% | - |

| 2. Dry Combing | | | | | |
|---|---|---|---|---|---|
| | Poor | Fair | Moderate | Good | Excellent |
| Placebo | 50% | 30% | 15% | 5% | - |
| Example 1 | 15% | 10% | 30% | 40% | 5% |

| 3. Feel of Hair Texture | | | | | |
|---|---|---|---|---|---|
| | Very Soft | Soft | Medium | Stiff | Very Stiff |
| Placebo | | 5% | 50% | 35% | 10% |
| Example 1 | 5% | 65% | 15% | 10% | 5% |

| 4. Cleaning Effect | | | | | |
|---|---|---|---|---|---|
| | Poor | Fair | Moderate | Good | Excellent |
| Placebo | 15% | 25% | 20% | 40% | - |
| Example 1 | 5% | - | 30% | 50% | 15% |

| 5. Manageability | | | | | |
|---|---|---|---|---|---|
| | Poor | Fair | Moderate | Good | Excellent |
| Placebo | 30% | 45% | 20% | 5% | - |
| Example 1 | - | 15% | 55% | 25% | 5% |

6. Hair Breakage

Panel members were asked to give the extent of their agreement with the statement "reduces hair breakage".

| | Strongly disagree | Slightly disagree | Neither agree nor disagree | Slightly agree | Strongly agree |
|---|---|---|---|---|---|
| Placebo | 30% | 25% | 40% | 5% | - |
| Ex. 1 | - | 15% | 30% | 50% | 5% |

It can be seen from these results that washing the hair with the composition of Example 1 gives improved results in wet and dry combing, feel, cleaning effect and overall manageability when compared with the placebo composition containing no reducing agent.

Furthermore, 55% of panellists using the composition of Example 1 agree (slightly or strongly) that the composition of Example 1 reduces hair breakage, whereas only 5% of panellists using the placebo agree that it reduces hair breakage.

| Example 2 | |
| --- | --- |
| | %w/w |
| JAGUAR C13S | 1.0 |
| Sodium hydroxide | 0.2 |
| Lactic acid | 0.5 |
| Triethanolamine lauryl sulphate | 30.0 |
| GAFQUAT 755N | 2.0 |
| Urea | 16.0 |
| Ammonium bisulphite | 8.325 |
| Ammonium sulphite | 2.0 |
| Ammonium hydroxide (32%) | 0.875 |
| Perfume | qs |
| Dye | qs |
| Water | to 100 |

A. Salon Test

The shampoo formulation of Example 2 was tested against a commercially available product in a hair salon.

Test Method

Sixteen panellists having untreated "virgin negroid hair" took part in a comparison test, performed by a professional hairdresser.

The products tested were

Shampoo A Shampoo of Example 2

Shampoo B Commercially available shampoo for permed hair

Conditioner C Sodium bromate rinse-off conditioner

Conditioner D Commercially available conditioner for permed hair

The commercially availbale products are formulated for use on permed hair and as such are mild and highly conditioning. They are also expected to impart some benefit to damaged hair.

The test was conducted on a daily basis for a period of 3 weeks. The panellists were divided into 4 groups, and each panellist was shampooed daily, Groups 1 and 2 for 1 minute and Groups 3 and 4 for 5 minutes. On every 5th day, panellists in Groups 2 and 4 were given conditioning treatments (see below).

The hair was sectioned in two and one half was shampooed with Shampoo A and the other half was shampooed with Shampoo B. Conditioning treatments were carried out using Conditioner C (on hair shampooed with Shampoo A) and Conditioner D (on hair shampooed with Shampoo B).

Neither the panellist nor the hairdresser was aware of which product was being applied to which half-head.

The professional hairdresser was asked to score for ease of combing (1-poor to 5-excellent) and hair breakage (1-none to 5-severe), and the results are summarised as follows:

1. Ease of Combing

The results below are the combined scores for 4-good and 5-excellent, expressed as a percentage of the total number of heads tested.

| | Shampoo A | Shampoo B | Probability |
|---|---|---|---|
| Group 1 | 81.7 | 68.3 | >95% |
| Group 2 | 71.7 | 40.0 | >99% |
| Group 3 | 48.9 | 11.1 | >99% |
| Group 4 | 88.1 | 32.2 | >99% |

2. Hair breakage

The results below are the combined scores for 1-none and 2-slight, expressed as a percentage of the total number of heads tested.

| | Shampoo A | Shampoo B | Probability |
|---|---|---|---|
| Group 1 | 98.3 | 93.3 | <95% |
| Group 2 | 88.3 | 78.3 | <95% |
| Group 3 | 91.1 | 73.3 | <95% |
| Group 4 | 86.4 | 67.8 | ±95% |
| Group 1 1 minute shampooing | | | |
| Group 2 1 minute shampooing and conditioning | | | |
| Group 3 5 minute shampooing | | | |
| Group 4 5 minute shampooing and conditioning | | | |

Discussion

The results set out above show that Shampoo A (the shampoo of the invention) gives better results for both ease of combing and hair breakage than Shampoo B (commercially available shampoo for permed hair) for all 4 groups.

Generally 1 minute contact time gives better results than 5 minute contact time. This is thought to be due to the preferential treatment of the weak spots on the hair shaft. These weak spots, caused by a twist or kink in the hair shaft, have a collapsed structure which will swell relatively quicker and so allow for a relatively quicker penetration of the reducing agent. Longer contact times give more generalised reduction, and can lead to damage of the hair shaft giving increased levels of hair breakage and poor combing effects.

The results also show that use of a conditioner comprising neutralising agents (Conditioners C and D) reduces the ease of combing and causes a slight increase in hair breakage for the one minute contact time (Groups 1 and 2).

B. Instron Test

The shampoo formulation of Example 2 was tested using the instron test method described below.

Method

A split-fibre technique was used in order to eliminate discrepancies due to varying diameter along the hair shaft. Caucasian-type hair fibres were tested since it is difficult to obtain sufficient numbers of negroid-type hair fibres of a reasonable length. Caucasian-type fibres are also easier to manipulate than Negroid-type fibres since they are less prone to breakage. Although the gross fibre structure may be different between the two hair types, their chemical composition is very similar.

The hair fibres used were 20cm long. One end was treated, the other end untreated (control). Two groups of hair were treated; one group had the root end as control, the tip end treated; the other group had the tip end as control, the root end being treated. This was done to cancel any discrepancies with regards to varying strength from root to tip.

The shampoo of Example 2 was applied to the treated end for 5 minutes, the amount of product used being one-tenth the weight of the hair swatch. Care was taken to wash the hair in a root-to-tip direction, to avoid abrasion of the cuticle scales.

To avoid tangling of hair and to ensure even distribution of product, the product was combed through (root-to-tip direction) at the relevant ends. After 5 minutes, the hair was rinsed thoroughly under running water for 2 minutes, blotted dry with absorbent paper and air dried for 2 hours. This treatment was repeated for the required number of times (up to a maximum of 50) the treatment regime being:

Untreated
1 treatment
5 treatments
10 treatments
20 treatments
50 treatments

Tensile strength tests, starting with the untreated hair, were done after each set of treatments. Approximately 40 hair strands were isolated after each treatment regime. The strands were kept at 52% Relative Humidity for 18 hours prior to testing.

The hair strands were then cut into two halves, testing the control end and the corresponding treated end on the Instron Tensile tester. The resulting stress/strain curve gave values for the Hookean Region, Yield Region and the post-yield region as described in the article by Dr W Cannell and L E Carothers, J.Soc.Cosmet.Chem.(1978) Vol. 29, pages 685-701. The value of the post-yield slope (PYS) of the treated end was subtracted from the PYS value of the control and expressed as a percentage of the control.

Post-yield slope is directly related to the disulphide bonding in the hair fibre.

The difference in post-yield slope between the control and treated ends are set out in the Table below.

| No. of treatments | n | $\bar{x}$ |
|---|---|---|
| 0 | 23 | - 3.14 |
| 1 | 37 | - 4.44 |
| 5 | 42 | 14.33 |
| 10 | 42 | 15.56 |
| 20 | 40 | 7.63 |
| 50 | 40 | 7.79 |

where n = number of hair fibres,
and $\bar{x}$ = sample mean of differences between PYS values of control and treated ends.

A large value of $\bar{x}$ indicates a greater degree of cleavage of the disulphide bonds in the hair fibre, and a positive value of $\bar{x}$ indicates a reduction in PYS for the treated end. It can be seen from the Table that the number of intact disulphide bonds in the hair tends to decrease with the number of treatments with the shampoo of the invention, until a certain point at between 10 and 20 repeats. After that point the benefit obtained appears to stabilise.

Example 3

The following formulation is suitable for virgin hair (ie. untreated hair).

| | % w/w |
|---|---|
| LAMEQUAT L | 3.0 |
| Urea | 16.0 |
| Ammonium bisulphite | 8.3 |
| Ammonium sulphite | 2.0 |
| Ammonium hydroxide (32%) | 0.875 |
| Cocamidopropyl betaine | 8.0 |
| Sodium laureth sulphate | 5.0 |
| Perfume | 0.5 |
| Preservative | 0.01 |
| Water | to 100 |
| pH 6.9 | |

Example 4

The following formulation is suitable for damaged or treated hair:

| | % w/w |
|---|---|
| Urea | 13.5 |
| Ammonium bisulphite | 6.3 |
| Ammonium sulphite | 1.5 |
| Ammonium hydroxide (32%) | 0.5 |
| Cocamidopropyl betaine | 10.0 |
| CROQUAT WKP | 2.0 |
| Perfume | 0.5 |
| Preservative | 0.01 |
| Water | to 100 |

CROQUAT WKP (trade name) is hydrolysed keratin protein containing sulphur bearing amino acids.

Example 5

The following is an example of a soap bar according to the invention.

| | %w/w |
|---|---|
| Sodium soap anhydrous:<br>fatty acid 50.77<br>sodium hydroxide 4.23 | 55.0 |
| Titanium dioxide | 0.3 |
| EDTA | 0.02 |
| Ethane hydroxydiphosphonic acid | 0.019 |
| Phosphoric acid | 0.215 |
| Urea | 12.0 |
| Ammonium bisulphite | 5.0 |
| Ammonium sulphite | 1.25 |
| Ammonium hydroxide (32%) | 0.50 |
| GAFQUAT 755N | 2.0 |
| Sodium chloride | 1.0 |
| Perfume | qs |
| Water | to 100 |

## Claims

1. An aqueous hair treatment composition for negroid hair which comprises
(a) detergent active compound chosen from anionic, nonionic and amphoteric detergent active compounds or mixtures thereof;
(b) cationic conditioning agent; and
(c) reducing agent capable of breaking disulphide bonds in the hair.

2. An aqueous hair treatment composition according to Claim 1 wherein the detergent active compound comprises from 2 to 40% by weight of the composition.

3. An aqueous hair treatment composition according to Claim 1 or 2 wherein the cationic conditioning agent is chosen from cationic detergent active compound, cationic polymers, cationic protein derivatives or mixtures thereof.

4. An aqueous hair treatment composition according to Claim 1, 2 or 3 wherein the cationic conditioning agent comprises from 0.001 to 10% by weight of the composition.

5. An aqueous hair treatment composition according to any preceding Claim wherein the reducing agent is chosen from thiols, mercaptans, sulphites, bisulphites or mixtures thereof.

6. An aqueous hair treatment composition according to Claim 5 wherein the reducing agent is a mixture of sulphite and bisulphite.

7. An aqueous hair conditioning composition according to any preceding Claim wherein the reducing agent comprises from 0.1 to 15% by weight of the composition.

8. An aqueous hair treatment composition according to any preceding claim wherein the composition is in the form of a shampoo.

9. An aqueous hair treatment composition according to Claims 1 to 7 wherein the composition is in the form of a bar product.

European Patent Office

# EUROPEAN SEARCH REPORT

Application number

EP 90304332.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB - A - 1 160 235 (L'OREAL) * Claims 28-34; page 2, lines 71-113 * | 1,2,5, 7,8 | A 61 K 7/075<br>A 61 K 7/06 |
| X | US - A - 4 295 985 (H.G.PETROW et al.) * Abstract; column 1, lines 64-68; column 4, line 64 - column 5, line 22; column 9, lines 30-34 * | 1,3, 5-8 | |
| Y | US - A - 4 465 619 (J.V.BOSKAMP) * Column 1, line 60 - column 3, line 16 * | 1-8 | |
| Y | EP - A2 - 0 277 876 (LABORATOIRE LACHARTRE SOC.) * Abstract; page 2, lines 53-58; page 5, lines 7-19 * | 1-8 | |

TECHNICAL FIELDS SEARCHED (Int Cl.5)

A 61 K 7/00

The present search report has been drawn up for all claims

| Place of search | Date of completion of the search | Examiner |
|---|---|---|
| VIENNA | 23-07-1990 | IRMLER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document